Europäisches Patentamt

European Patent Office

Office européen des brevets

(19)

(11) **EP 1 410 804 A1**

(12) **EUROPEAN PATENT APPLICATION**
published in accordance with Art. 158(3) EPC

(43) Date of publication:
**21.04.2004 Bulletin 2004/17**

(51) Int Cl.⁷: **A61K 39/00**, A61K 45/08,
A61P 35/00, A61P 35/02

(21) Application number: **02738876.8**

(22) Date of filing: **28.06.2002**

(86) International application number:
**PCT/JP2002/006597**

(87) International publication number:
**WO 2003/002142 (09.01.2003 Gazette 2003/02)**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **29.06.2001 JP 2001199449**

(71) Applicants:
• **CHUGAI SEIYAKU KABUSHIKI KAISHA
Tokyo, 115-8543 (JP)**
• **Mayumi, Tadanori
Koube-shi, Hyogo 655-0041 (JP)**
• **Sugiyama, Haruo
Mino-shi, Osaka 562-0036 (JP)**

(72) Inventors:
• **MAYUMI, Tadanori
Koube-shi, Hyogo 655-0041 (JP)**
• **SUGIYAMA, Haruo
Minoo-shi, Osaka 562-0036 (JP)**
• **OHSUGI, Yoshiyuki, c/o Chugai Seiyaku K. K.
Tokyo 104-8301 (JP)**

(74) Representative: **Holliday, Louise Caroline
D Young & Co,
21 New Fetter Lane
London EC4A 1DA (GB)**

(54) **CANCER VACCINE CONTAINING CANCER ANTIGEN BASED ON TUMOR SUPPRESSOR GENE WT1 PRODUCT AND CATIONIC LIPOSOMES**

(57)  A cancer vaccine comprising a cancer antigen which comprises, as an active ingredient, the product of a tumor suppressor gene WT1, a partial peptide or a modified version thereof, and a cationic liposome.

**EP 1 410 804 A1**

**Description**

Field of the Invention

The present invention relates to a cancer vaccine comprising a cancer antigen based on the product of a tumor suppressor gene WT1 of Wilms tumor and lipofectin. This cancer vaccine is useful as an anti-cancer vaccine for blood cancers such as leukemia, myelodysplastic syndrome, multiple myeloma and malignant lymphoma, or solid cancers such as gastric cancer, colon cancer, lung cancer, breast cancer, germ cell cancer, liver cancer, skin cancer, bladder cancer, prostatic cancer, uterine cancer, cervical cancer and ovarian cancer, as well as all other cancers that express WT1.

Background Art

Immunological mechanisms for eliminating foreign substances generally comprise the humoral immunity which involves macrophages that recognize antigen so as to function as antigen presenting cells, helper T cells that recognize the antigen presentation by said macrophages and produce various lymphokines so as to activate other T cells etc., and B lymphocytes, etc., that differentiate into antibody-producing cells by the action of said lymphokines; and the cellular immunity in which killer T cells differentiated by antigen presentation attack and destroy target cells.
At present, cancer immunity is mainly derived from cellular immunity which involves killer T cells. In killer T cell-mediated cancer immunity, precursor T cells that recognized cancer antigen presented in the form of a complex with the major histocompatibility complex (MHC) class I differentiate and grow to produce killer T cells, which attack and destroy cancer cells. At this time, cancer cells have presented the complex of the MHC class I antigen and cancer antigen on the cell surface, which becomes the target for killer T cells (Curr. Opin. Immuno. 5:709, 1993; Curr. Opin. Immunol. 5:719, 1993; Cell 82:13, 1995; Immunol. Rev. 146:167, 1995).
The above cancer antigen presented on the target cancer cells by MHC class I antigen is believed to be a peptide composed of about 8-12 amino acids produced as a result of processing by intracellular protease of antigen proteins synthesized in the cancer cells (Curr. Opin. Immunol. 5:709, 1993; Curr. Opin. Immunol. 5:719, 1993; Cell 82: 13, 1995; Immunol. Rev. 146:167, 1995).
The tumor suppressor gene WT1 (WT1 gene) of Wilms tumor was isolated from chromosome 11p13 as one of the causative genes for Wilms tumor based on the analysis of the WAGR syndrome that is accompanied by Wilms tumor, aniridia, urogenital abnormality, mental retardation etc. (Gessler, M. et al., Nature, Vol. 343, pp. 774-778, 1990), and its genomic DNA is about 50 kb comprising ten exons and its cDNA is about 3 kb. The amino acid sequence deduced from the cDNA is as set forth in SEQ ID NO: 1 (Mol. Cell. Biol. 11:1707, 1991).
The WT1 gene is highly expressed in human leukemia, and the treatment of leukemic cells with a WT1 antisense oligomer leads to the suppression of the cell growth (Japanese Unexamined Patent Publication (Kokai) No. 9-104627), which suggests that the WT1 gene is acting on the growth of leukemic cells in a facilitative manner. Furthermore, the WT1 gene has also been highly expressed in solid cancers such as gastric cancer, colon cancer, lung cancer, breast cancer, germ cell cancer, liver cancer, skin cancer, bladder cancer, prostatic cancer, uterine cancer, cervical cancer and ovarian cancer (Japanese Patent Application No. 9-191635), and the WT1 gene was found to be a new tumor marker for leukemia and solid cancers.
Thus, it is expected that the administration of a peptide having about 8-12 amino acids comprising a portion of expression products of the WT1 gene could serve as a cancer vaccine against the above range of cancers. However, the administration of such a peptide as it is cannot serve as a cancer vaccine. This is because it is expected that the peptide administered cannot be effectively delivered to the major histocompatibility complex class I on the antigen-presenting cells.
Lipofectin, a cationic liposome, is a 1:1 mixture of an artificial lipid N-[1-(2,3-dioleyloxy)propyl]-N,N,N-trimethylammonium chloride (DOTMA) and a phospholipid dioleoylphosphatidylethanolamine (DOPE), and attracted attention as a nonviral carrier for introducing genes. Subsequently, attention was given to the fact that it can serve to deliver peptide antigens to the major histocompatibility complex class I on the antigen-presenting cells (Rinsho Menneki 34 (6):842-847, 2000). However, the degree of versatility of cationic liposomes as carriers for peptide antigens is unknown, and it is not known either whether they can serve as carriers for cancer antigen peptides comprising fragments of expression products of the tumor suppressor gene WT1 gene.

Disclosure of the Invention

Thus, the present invention provides a novel cancer vaccine comprising a cancer antigen peptide derived from a WT1 gene expression product and a substance useful as a carrier therefor.
After intensive and extensive research in order to solve the above problems, the present inventors have confirmed that, in the amino acid sequence of expression product of the WT1 gene, a polypeptide comprising 7-30 contiguous amino acids containing at least one amino acid that is estimated to serve as an anchor amino acid serves as a cancer antigen in the binding with the mouse and human MHC class I and MHC class II, and that cationic liposomes such as lipofectin are useful as carriers for this peptide antigen and, thereby, have completed the present invention.

Thus, the present invention provides a cancer vaccine comprising a cancer antigen containing a mouse WT1 gene expression product or a portion thereof, and a cationic liposome. In a preferred embodiment, the present invention provides a cancer vaccine comprising a cancer antigen that comprises as an active ingredient a peptide comprising 6-30 amino acids containing at least one amino acid selected from the group consisting of Phe, Tyr, Leu, Met, Asn and Ile, that is estimated to function as an anchor amino acid for binding to the MHC antigen, in the amino acid sequence as set forth in SEQ ID NO: 1 corresponding to the cDNA of the MHC antigen, and a cationic liposome.

Furthermore, the present invention provides a cancer vaccine comprising a cancer antigen that comprises as an active ingredient a peptide comprising 7-30 amino acids containing at least one amino acid selected from the group consisting of Met, Leu, and Val, that is estimated to function as an anchor amino acid for binding to the MHC antigen, in an amino acid sequence as set forth in SEQ ID NO: 2 corresponding to the cDNA of human WT1, and a cationic liposome.

Brief Explanation of the Drawings

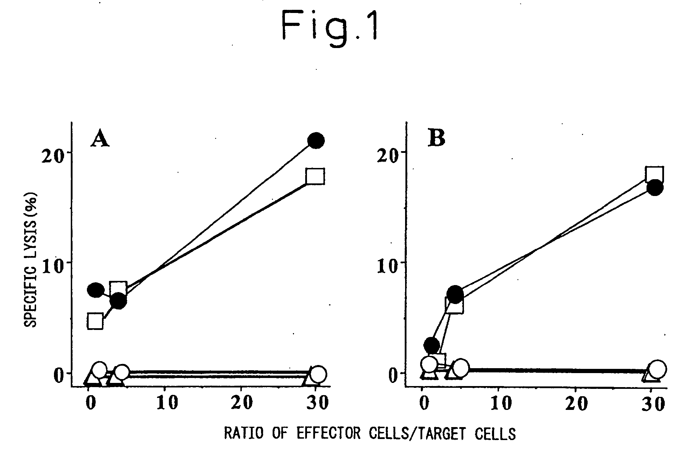

In Fig. 1, A is a graph that compares the ability of inducing cytotoxic T cells of a mixture (closed circle) of a cancer antigen peptide $D^b$ 126 and lipofectin (LPF), a lipopolysaccharide-blast (open square) pulsed with $D^b$ 126, lipofectin alone (open triangle) and the cancer antigen peptide $D^b$ 126 alone (open circle) using (3) RNAS cells and (4) RNAS cells stimulated with the cancer antigen peptide $D^b$ 126, and B is a graph of the result in which tests similar to the above A were carried out using (1) C1498 cells and (2) WT1 gene-introduced C1498 cells. A indicates that the combination of the cancer antigen peptide $D^b$ 126 and lipofectin has an activity of inducing cytotoxic T cells, and B indicates that the activity thereof is WT1-specific.

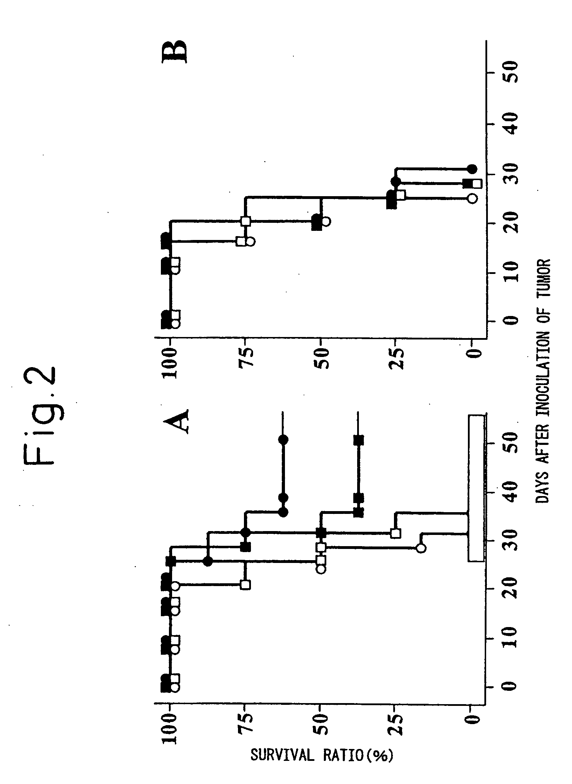

In Fig. 2, A is a graph that shows the effect of lipofectin as an adjuvant (carrier) for the anti-cancer effect of the peptide $D^b$ 126 using WT1 gene-introduced C1498 cells, and B is a graph that shows the result of similar tests using C1498 cells. Signs that indicate the test substances are the same as in Fig. 1. A indicates that lipofectin is effective as an adjuvant (carrier) for the cancer antigen peptide $D^b$ 126, and the comparison of A and B shows that the anti-cancer effect is WT1-specific.

Embodiment for Carrying Out the Invention

In accordance with the present invention, as a basis for designing cancer antigen peptides, $K^b$ and $D^b$ of mouse MHC class I as well as A0201 of human HLA were selected, and peptides estimated to have a high affinity with them were selected.

Based on the description in Immunogenetics 41:178-228 (1995), Phe and Try at position 5 as well as Leu and Met etc. at position 8 are expected to be the anchor amino acids for binding to $K^b$, and Asn at position 5 as well as Met and Ile etc. at position 9 are expected to be the anchor amino acids for binding to $D^b$.

It is also known that the size of cancer antigen peptides presented on the surface of cancer cells by MHC class I is about 8-12 amino acids. Thus, the cancer antigen peptide of the present invention is a peptide comprising 7-30 contiguous amino acids containing at least one amino acid of Phe, Tyr, Leu, Met, Asn and Ile in the amino acid sequence of the WT1 gene product as set forth in SEQ ID NO: 1. The number of amino acids is preferably 8-12, for example 8 or 9.

In accordance with the present invention, specific embodiments include, as a peptide that binds to $K^b$ of MHC class I, the following peptides comprising 8 amino acids:

$K^b$ 45 Gly Ala Ser Ala Tyr Gly Ser Leu (SEQ ID NO: 3)

$K^b$ 330 Cys Asn Lys Arg Tyr Phe Lys Leu (SEQ ID NO: 4), and,

as a peptide that binds to $D^b$ of MHC class I, the following peptides comprising 9 amino acids:

```
D^b 126   Arg Met Phe Pro Asn Ala Pro Tyr Leu (SEQ ID
NO: 5)
```

```
D^b 221   Tyr Ser Ser Asp Asn Leu Tyr Gln Met (SEQ ID
NO: 6)
```

```
D^b 235   Cys Met Thr Trp Asn Gln Met Asn Leu (SEQ ID
NO: 7).
```

In the above sequences, the underlined amino acids are those that are thought to serve as anchors.

All of them have strong to moderate binding affinities (Kd values) for $K^b$ or $D^b$, and the $D^b$ 126 peptide having the highest binding affinity was used in the following experiments.

For humans, based on the description in Immunogenetics 41:178-228 (1995), Leu and Met at position 2 from the N-terminal and Val and Leu at position 9 from the N-terminal are expected to be anchor amino acids for binding to human HLA-A0201. Thus, from among the amino acid sequence of human WT1 protein (Mol. Cell. Biol. 11:1707-1712, 1991) (SEQ ID NO: 2), the following two peptides:

```
D^b 126   Arg Met Phe Pro Asn Ala Pro Tyr Leu (SEQ ID
NO: 5)
```

(the same as $D^b$ 126 in mice)

```
WH 187   Ser Leu Gly Glu Gln Gln Tyr Ser Val (SEQ ID
NO: 8)
```

(the underlined are anchor amino acids)

comprising nine amino acids are mentioned as complying with the above condition.

The cancer antigen peptide of the present invention may also be a peptide in which a modification such as amino acid substitution has been introduced into a peptide which is a portion of the expression product of the WT1 gene. As an example of such a modified peptide, there can be mentioned a cancer antigen peptide comprising as an active ingredient a peptide that comprises 9-30 amino acids containing the following amino acid sequence: Cys Tyr Thr Trp Asn Gln Met Asn Leu (SEQ ID NO: 9). As a specific embodiment, there can be mentioned a peptide having an amino acid sequence: Cys Tyr Thr Trp Asn Gln Met Asn Leu (SEQ ID NO: 9) in which Met at position 2 of the above peptide $D^b$ 235 (SEQ ID NO: 7) has been changed to Tyr.

As a cationic liposome, there can be mentioned a liposome comprising N-[1-(2,3-dioleyloxy)propyl]-N,N,N-trimethylammonium chloride (DOTMA), N-[1-(2,3-dioleyloxy)propyl]-N,N,N-trimethylammonium methyl sulfate (DOTAP) or dioctadecylamide-glycylspermine (DOGS), or mixtures thereof with a neutral lipid.

As a neutral lipid, there can be mentioned, for example, licithin, lysolecithin, sphingomyelin, phosphatidic acid, phosphatidylethanolamine, and dioleoylphosphatidylethanolamine (DOPE). As an example of mixtures, there can be mentioned lipofectin which is a 1:1 mixture of an artificial lipid N-[1-(2,3-dioleyloxy)propyl]-N,N,N-trimethylammonium chloride (DOTMA) and a phospholipid dioleoylphosphatidylethanolamine (DOPE).

The cancer vaccine of the present invention can be used for the prevention or treatment of cancers that are accompanied by increased levels of the WT1 gene expression, for example blood cancers such as leukemia, myelodysplastic syndrome, multiple myeloma and malignant lymphoma, and solid cancers such as gastric cancer, colon cancer, lung cancer, breast cancer, germ cell cancer, liver cancer, skin cancer, bladder cancer, prostatic cancer, uterine cancer, cervical cancer and ovarian cancer. This vaccine can be administered by oral administration, or parenteral administration such as intraperitoneal, cutaneous, dermal, intramuscular, intravenous, and nasal administration.

The dosage of the cancer vaccine of the present invention is generally 0.1 μg to 1 mg/kg per day.

Examples

The usefulness of the cancer vaccine of the present invention will now be explained with reference to Examples.

Example 1.

Preparation of lipopolysaccharide-blast (LPS-blast)

From C57BL/6 mice, spleen cells were recovered, and the cells were incubated for 3 days in a complete RPMI medium containing lipopolysaccharide (LPS) (10 µg/ml). After washing, the cells were incubated in a complete RPMI medium containing the cancer antigen peptide $D^b$ 126 (1 µM) and ovalbumin (OVA) (100 µg/ml). After washing, the cells were suspended in 2 ml Hanks' balanced salt solution (HBSS) which was set as the lipopolysaccharide-blast (LPS-blast).

Evaluation of the ability of inducing cytotoxic T cells (CTL)

C57BL/6 mice were immunized three times weekly by subcutaneous administration, to the back thereof, of a mixture of the cancer antigen peptide $D^b$ 126 and lipofectin (LPF) (mixed at a 1:2 weight ratio of $D^b$ 126 and LPF), and as a positive control by the intraperitoneal administration of lipopolysaccharide-blast (LPS-blast) (1 ml/mouse). Ten days after the final immunization, spleen cells were recovered and set as effector cells. Spleen cells stimulated with the cancer antigen peptide $D^b$ 126 (1 µM, 2 hours, 37°C, 5% CO2) were washed with HBSS to obtain stimulator cells.
The above effector cells (5 x 106 cells/well) and the above stimulator cells (2.5 x 106 cells/well) were mixed, and then subjected to lymphocyte-lymphocyte mixed culture for the in vitro second challenge of the cytotoxic T cells. Five days later, cytotoxic T cells were recovered. (1) C1498 cells, (2) C1498 cells that have introduced therein and express the WT1 gene (C1498muWT1), (3) RMA-S cells, and (4) RMA-S cells stimulated with the cancer antigen $D^b$ 126 (1 µM, treated for 1 hour at 5% CO2) ($D^b$ 126-pulsed RMA-S), each labelled with $Na_2{}^{51}CrO_4$ (0.56 MBq/$10^6$ cells, treated for 1 hour at 37°C and 5% CO2), were plated as the target cells onto 96-well microtiter plates ($10^4$ cells/well), to which cytotoxic T cells prepared as above were plated. Effector cells were added thereto, cultured for 4 hours, and the radioactivity of $^{51}Cr$ liberated into the supernatant was counted. Cytotoxic activity was calculated according to the following equation:

$$\text{Cell lysis (\%)} = \frac{\text{(experimental release - spontaneous release)}}{\text{(maximum release - spontaneous release)}} \times 100$$

Results

First, in order to examine whether or not the induced cytotoxic T cells are specific for the cancer antigen peptide $D^b$ 126, the above (3) RMA-S cells and (4) $D^b$ 126-pulsed RMA-S cells were used as the target cells, and as a result the induction of cytotoxic T cells specific for the cancer antigen peptide $D^b$ 126 was confirmed (Fig. 1, A). Furthermore, in order to examine whether or not the induced cytotoxic T cells specifically damage WT1-expressing cells, the above (1) C1498 cells and (2) the WT1 gene-introduced cells, C1498muWT1, were used, and as a result the induction of WT1-specific CTL was confirmed (Fig. 1, B).

Example 2.

Cancer antigen-specific anti-tumor effect when lipofectin (LPF) was used as the cancer vaccine carrier

Since Example 1 has shown that cytotoxic T cells are effectively induced by using lipofectin (LPF) as an adjuvant for the cancer antigen peptide $D^b$ 126, cancer antigen-specific anti-tumor effect when immunized using lipofectin as an adjuvant (carrier) was examined for the purpose of further confirming the usefulness of lipofectin (LPF) as an adjuvant for cancer vaccines.
As the tumor model, WT1 gene-introduced C1498 cells (C1498muWT1 cells) were used; as the immunization animal, C57BL/6 mice were used; and as the model cancer antigen, the peptide $D^b$ 126 was used. Thus, C57BL/6 mice were immunized three times weekly by subcutaneous administration, to the back thereof, of the same mixture as in Example 1 of the cancer antigen peptide $D^b$ 126 and lipofectin (LPF) (10 nmol/mouse), or by the intraperitoneal administration of lipopolysaccharide-blast (LPS-blast) (1 ml), and one week after the final immunization C1498muWT1 cells or C1498 cells were intraperitoneally transplanted at an amount of $2 \times 10^6$ cells/100 ml. The effect of tumor vaccine was determined daily, and was evaluated by calculating tumor size using the following equation:

$$[\text{Tumor size}] = [(\text{long diameter}) \times (\text{short diameter})^2]^{1/3}$$

In each group, the experiment was terminated when tumor size reached 20 mm.

Results

The evaluation of lipofectin (LPF) as an adjuvant (carrier) for cancer vaccine was carried out using WT1 as the model tumor antigen and WT1 gene-introduced cells (C1498muWT1 cells) as the model tumor, and using, as an index, resistance against C1498muWT1 cells when the peptide $D^b$ 126/lipofectin (LPF) mixture was used for immunization. As a result, when the peptide $D^b$ 126/lipofectin (LPF) mixture was used for immunization, complete rejection was observed in three out of eight cases (Fig. 2, A).

Furthermore, in order to confirm that this anti-tumor effect is WT1-specific, a similar study was carried out using C1498 cells that are not expressing WT1. As a result, there were no differences seen from the non-immunized group in any of the cases in which (a) peptide $D^b$ 126/lipofectin (LPF) mixture, (b) free peptide $D^b$ 126, and (c) lipopolysaccharide-blast (LPS-blast) were immunized (Fig. 2, B). Therefore, it was confirmed that the above anti-tumor effect is WT1-specific.

**Claims**

1. A cancer vaccine comprising a cancer antigen which comprises as an active ingredient the product of a tumor suppressor gene WT1, a partial peptide or a modified version thereof, and a cationic liposome.

2. The cancer vaccine according to claim 1 wherein said cancer antigen is a peptide comprising 7-30 contiguous amino acids containing at least one anchor amino acid selected from Phe, Tyr, Leu, Met, Asn and Ile in the amino acid sequence as set forth in SEQ ID NO: 1, or a peptide comprising 7-30 contiguous amino acids containing at least one anchor amino acid selected from Met, Leu and Val in the amino acid sequence as set forth in SEQ ID NO: 2.

3. The cancer vaccine according to claim 1 or 2 wherein said antigen is a cancer antigen that permits a high expression of the tumor suppressor gene WT1.

4. The cancer vaccine according to claim 1 or 2 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

5. The cancer vaccine according to any of claims 1 to 4 wherein said cancer antigen peptide is any of:

```
        Kb   45   Gly Ala Ser Ala Tyr Gly Ser Leu (SEQ ID
NO: 3)


        Kb 330   Cys Asn Lys Arg Tyr Phe Lys Leu (SEQ ID
NO: 4)


           Db 126   Arg Met Phe Pro Asn Ala Pro Tyr Leu
  (SEQ ID NO: 5)


        Db 221   Tyr Ser Ser Asp Asn Leu Tyr Gln Met
  (SEQ ID NO: 6)


        Db 235   Cys Met Thr Trp Asn Gln Met Asn Leu
  (SEQ ID NO: 7), and
```

```
WH 187  Ser Leu Gly Glu Gln Gln Tyr Ser Val
```


```
                     (SEQ ID NO: 8).
```

6.  The cancer vaccine according to claim 5 wherein said cancer antigen peptide is:


```
    Dᵇ 126  Arg Met Phe Pro Asn Ala Pro Tyr Leu
(SEQ ID NO: 5), or
```


```
    WH 187  Ser Leu Gly Glu Gln Gln Tyr Ser Val
(SEQ ID NO: 8).
```

7.  The cancer vaccine according to claim 1 wherein said cancer antigen peptide is a peptide comprising 9-30 amino acids containing the following amino acid sequence: Cys Tyr Thr Trp Asn Gln Met Asn Leu (SEQ ID NO: 9).

8.  The cancer vaccine according to claim 7 wherein said cancer antigen peptide is a peptide comprising the following amino acid sequence: Cys Tyr Thr Trp Asn Gln Met Asn Leu (SEQ ID NO: 9).

9.  The cancer vaccine according to any one of claims 1 to 8 wherein said cationic liposome is a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

10. The cancer vaccine according to claim 9 wherein said cationic liposome is lipofectin.

11. The use of a cancer antigen which comprises as an active ingredient the product of the tumor suppressor gene WT1, a partial peptide or a modified version thereof, and a cationic liposome for the production of cancer vaccine.

12. The use according to claim 11 wherein said cancer antigen is a peptide comprising 7-30 contiguous amino acids containing at least one anchor amino acid selected from Phe, Tyr, Leu, Met, Asn and Ile in the amino acid sequence as set forth in SEQ ID NO: 1, or a peptide comprising 7-30 contiguous amino acids containing at least one anchor amino acid selected from Met, Leu and val in the amino acid sequence as set forth in SEQ ID NO: 2.

13. The use according to claim 11 or 12 wherein said antigen is a cancer antigen that permits high expression of the tumor suppressor gene WT1.

14. The use according to claim 11 or 12 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

15. The use according to any of claims 11 to 14 wherein said cancer antigen peptide is any of:


```
    Kb  45  Gly Ala Ser Ala Tyr Gly Ser Leu (SEQ ID
NO: 3)
```


```
    Kb 330  Cys Asn Lys Arg Tyr Phe Lys Leu (SEQ ID
NO: 4)
```

```
                D  126   Arg Met Phe Pro Asn Ala Pro Tyr Leu
      (SEQ ID NO: 5)


                Db 221   Tyr Ser Ser Asp Asn Leu Tyr Gln Met
      (SEQ ID NO: 6)


                Db 235   Cys Met Thr Trp Asn Gln Met Asn Leu
      (SEQ ID NO: 7), and


                WH 187   Ser Leu Gly Glu Gln Gln Tyr Ser Val
      (SEQ ID NO: 8).
```

**16.** The use according to claim 15 wherein said cancer antigen peptide is:

```
                D  126   Arg Met Phe Pro Asn Ala Pro Tyr Leu
      (SEQ ID NO: 5), or


                WH 187   Ser Leu Gly Glu Gln Gln Tyr Ser Val
      (SEQ ID NO: 8).
```

**17.** The use according to claim 11 wherein said cancer antigen peptide is a peptide comprising 9-30 amino acids containing the following amino acid sequence: Cys Tyr Thr Trp Asn Gln Met Asn Leu (SEQ ID NO: 9).

**18.** The use according to claim 17 wherein said cancer antigen peptide is a peptide comprising the following amino acid sequence: Cys Tyr Thr Trp Asn Gln Met Asn Leu (SEQ ID NO: 9).

**19.** The use according to any one of claims 11 to 18 wherein said cationic liposome is a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

**20.** The use according to claim 19 wherein said cationic liposome is lipofectin.

**21.** A method of treating patients with cancer, said method comprising administering a cancer antigen which comprises as an active ingredient the product of the tumor suppressor gene WT1, a partial peptide or a modified version thereof, and a cationic liposome.

**22.** The method according to claim 21 wherein said cancer antigen is a peptide comprising 7-30 contiguous amino acids containing at least one anchor amino acid selected from Phe, Tyr, Leu, Met, Asn and Ile in the amino acid sequence as set forth in SEQ ID NO: 1, or a peptide comprising 7-30 contiguous amino acids containing at least one anchor amino acid selected from Met, Leu and Val in the amino acid sequence as set forth in SEQ ID NO: 2.

**23.** The method according to claim 21 or 22 wherein said antigen is a cancer antigen that permits high expression of the tumor suppressor gene WT1.

**24.** The method according to claim 21 or 22 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

**25.** The method according to any of claims 11 to 24 wherein said cancer antigen peptide is any of:

        Kb  45  Gly Ala Ser Ala Tyr Gly Ser Leu (SEQ ID NO: 3)

        Kb 330  Cys Asn Lys Arg Tyr Phe Lys Leu (SEQ ID NO: 4)

        D$^b$ 126  Arg Met Phe Pro Asn Ala Pro Tyr Leu (SEQ ID NO: 5)

        Db 221  Tyr Ser Ser Asp Asn Leu Tyr Gln Met (SEQ ID NO: 6)

        Db 235  Cys Met Thr Trp Asn Gln Met Asn Leu (SEQ ID NO: 7), and

        WH 187  Ser Leu Gly Glu Gln Gln Tyr Ser Val (SEQ ID NO: 8).

**26.** The method according to claim 25 wherein said cancer antigen peptide is:

        D$^b$ 126  Arg Met Phe Pro Asn Ala Pro Tyr Leu (SEQ ID NO: 5), or

        WH 187  Ser Leu Gly Glu Gln Gln Tyr Ser Val (SEQ ID NO: 8).

**27.** The method according to claim 21 wherein said cancer antigen peptide is a peptide comprising 9-30 amino acids containing the following amino acid sequence: Cys Tyr Thr Trp Asn Gln Met Asn Leu (SEQ ID NO: 9).

**28.** The method according to claim 27 wherein said cancer antigen peptide is a peptide comprising the following amino acid sequence: Cys Tyr Thr Trp Asn Gln Met Asn Leu (SEQ ID NO: 9).

**29.** The method according to any one of claims 21 to 28 wherein said cationic liposome is a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

**30.** The method according to claim 29 wherein said cationic liposome is lipofectin.

# Fig.1

# Fig.2

## INTERNATIONAL SEARCH REPORT

| International application No. |
|---|
| PCT/JP02/06597 |

| A. CLASSIFICATION OF SUBJECT MATTER |
|---|
| Int.Cl$^7$ A61K39/00, 45/08, A61P35/00, 35/02 |

According to International Patent Classification (IPC) or to both national classification and IPC

| B. FIELDS SEARCHED |
|---|
| Minimum documentation searched (classification system followed by classification symbols)<br>Int.Cl$^7$ A61K39/00, 45/08, A61P35/00, 35/02 |

Documentation searched other than minimum documentation to the extent that such documents are included in the fields searched

Electronic data base consulted during the international search (name of data base and, where practicable, search terms used)
WPI(DIALOG), BIOSIS(DIALOG), CAS(STN), MEDLINE(STN), DDBJ/EMBL/GenBank/Geneseq

### C. DOCUMENTS CONSIDERED TO BE RELEVANT

| Category* | Citation of document, with indication, where appropriate, of the relevant passages | Relevant to claim No. |
|---|---|---|
| Y | G. Alexander, 'Immunity to WT1 in the animal model and patients with acute myeloid leukemia', Vol.96, Blood, 2000, pages 1480 to 1489 | 1-20 |
| Y | Y. OKA, 'Human cytotoxic T-lymphocyte responses specific for peptides of the wild-type Wilms' tumor gene (WT1) product'. Vol.51, Immunogenetics, 2000, pages 99 to 107 | 1-20 |
| Y | G. Nakanishi, 'Kation-sei Liposome o kogen Carrier to shite Mochiita CTL yudo', Vol.34, Clinical Immunology, 2000, pages 842 to 847 | 1-20 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>17 September, 2002 (17.09.02) | Date of mailing of the international search report<br>08 October, 2002 (08.10.02) |
|---|---|
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.

PCT/JP02/06597

| Box I   Observations where certain claims were found unsearchable (Continuation of item 1 of first sheet) |
|---|

This international search report has not been established in respect of certain claims under Article 17(2)(a) for the following reasons:

1. ☒ Claims Nos.: 21-30

because they relate to subject matter not required to be searched by this Authority, namely:

The inventions as set forth in these claims include "treatments of the human body by surgery or therapy".

2. ☐ Claims Nos.:

because they relate to parts of the international application that do not comply with the prescribed requirements to such an extent that no meaningful international search can be carried out, specifically:

3. ☐ Claims Nos.:

because they are dependent claims and are not drafted in accordance with the second and third sentences of Rule 6.4(a).

| Box II   Observations where unity of invention is lacking (Continuation of item 2 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐ The additional search fees were accompanied by the applicant's protest.

☐ No protest accompanied the payment of additional search fees.

Form PCT/ISA/210 (continuation of first sheet (1)) (July 1998)

SEQUENCE LISTING

<110> Tadanori Mayumi et al.

<120> Cancer Vaccine Comprising a Cancer Antigen Based on the Product of a Tumor Suppressor Gene WT1 and a Cationic Liposome

<130> K807

<160> 9

<210> 1

<211> 449

<212> PRT

<213> Mouse

<400> 1

```
Met Gly Ser Asp Val Arg Asp Leu Asn Ala Leu Leu Pro Ala Val Ser
                    5                   10                  15

Ser Leu Gly Gly Gly Gly Gly Gly Cys Gly Leu Pro Val Ser Gly Ala
                   20                   25                  30

Arg Gln Trp Ala Pro Val Leu Asp Phe Ala Pro Pro Gly Ala Ser Ala
             35                   40                   45

Tyr Gly Ser Leu Gly Gly Pro Ala Pro Pro Pro Ala Pro Pro Pro Pro
         50                   55                   60

Pro Pro Pro Pro His Ser Phe Ile Lys Gln Glu Pro Ser Trp Gly Gly
    65                   70                   75                  80

Ala Glu Pro His Glu Glu Gln Cys Leu Ser Ala Phe Thr Leu His Phe
                     85                   90                  95

Ser Gly Gln Phe Thr Gly Thr Ala Gly Ala Cys Arg Tyr Gly Pro Phe
                   100                  105                  110

Gly Pro Pro Pro Pro Ser Gln Ala Ser Ser Gly Gln Ala Arg Met Phe
              115                  120                  125

Pro Asn Ala Pro Tyr Leu Pro Ser Cys Leu Glu Ser Gln Pro Thr Ile
        130                  135                  140

Arg Asn Gln Gly Tyr Ser Thr Val Thr Phe Asp Gly Ala Pro Ser Tyr
145                  150                  155                  160
```

7

Gly His Thr Pro Ser His His Ala Ala Gln Phe Pro Asn His Ser Phe
                165                 170                 175

Lys His Glu Asp Pro Met Gly Gln Gln Gly Ser Leu Gly Glu Gln Gln
                180                 185                 190

Tyr Ser Val Pro Pro Pro Val Tyr Gly Cys His Thr Pro Thr Asp Ser
                195                 200                 205

Cys Thr Gly Ser Gln Ala Leu Leu Leu Arg Thr Pro Tyr Ser Ser Asp
                210                 215                 220

Asn Leu Tyr Gln Met Thr Ser Gln Leu Glu Cys Met Thr Trp Asn Gln
225                 230                 235                 240

Met Asn Leu Gly Ala Thr Leu Lys Gly Met Ala Ala Gly Ser Ser Ser
                245                 250                 255

Ser Val Lys Trp Thr Glu Gly Gln Ser Asn His Gly Ile Gly Tyr Glu
                260                 265                 270

Ser Glu Asn His Thr Ala Pro Ile Leu Cys Gly Ala Gln Tyr Arg Ile
                275                 280                 285

His Thr His Gly Val Phe Arg Gly Ile Gln Asp Val Arg Arg Val Ser
                290                 295                 300

Gly Val Ala Pro Thr Leu Val Arg Ser Ala Ser Glu Thr Ser Glu Lys
305                 310                 315                 320

Arg Pro Phe Met Cys Ala Tyr Pro Gly Cys Asn Lys Arg Tyr Phe Lys
                325                 330                 335

Leu Ser His Leu Gln Met His Ser Arg Lys His Thr Gly Glu Lys Pro
                340                 345                 350

Tyr Gln Cys Asp Phe Lys Asp Cys Glu Arg Arg Phe Ser Arg Ser Asp
                355                 360                 365

Gln Leu Lys Arg His Gln Arg Arg His Thr Gly Val Lys Pro Phe Gln
                370                 375                 380

Cys Lys Thr Cys Gln Arg Lys Phe Ser Arg Ser Asp His Leu Lys Thr
385                 390                 395                 400

His Thr Arg Thr His Thr Gly Lys Thr Ser Glu Lys Pro Phe Ser Cys
                405                 410                 415

Arg Trp His Ser Cys Gln Lys Lys Phe Ala Arg Ser Asp Glu Leu Val
420                425                430

Arg His His Asn Met His Gln Arg Asn Met Thr Lys Leu His Val Ala
435                440                445

Leu

449

<210>    2

<211>    449

<212>    PRT

<213>    Human

<400>    2

Met Gly Ser Asp Val Arg Asp Leu Asn Ala Leu Leu Pro Ala Val Pro
                  5                10                15

Ser Leu Gly Gly Gly Gly Gly Cys Ala Leu Pro Val Ser Gly Ala Ala
                  20                25                30

Gln Trp Ala Pro Val Leu Asp Phe Ala Pro Pro Gly Ala Ser Ala Tyr
        35                40                45

Gly Ser Leu Gly Gly Pro Ala Pro Pro Ala Pro Pro Pro Pro Pro
    50                55                60

Pro Pro Pro Pro His Ser Phe Ile Lys Gln Glu Pro Ser Trp Gly Gly
 65                70                75                80

Ala Glu Pro His Glu Glu Gln Cys Leu Ser Ala Phe Thr Val His Phe
                  85                90                95

Ser Gly Gln Phe Thr Gly Thr Ala Gly Ala Cys Arg Tyr Gly Pro Phe
                  100                105                110

Gly Pro Pro Pro Pro Ser Gln Ala Ser Ser Gly Gln Ala Arg Met Phe
        115                120                125

Pro Asn Ala Pro Tyr Leu Pro Ser Cys Leu Glu Ser Gln Pro Ala Ile
        130                135                140

Arg Asn Gln Gly Tyr Ser Thr Val Thr Phe Asp Gly Thr Pro Ser Tyr
145                150                155                160

Gly His Thr Pro Ser His His Ala Ala Gln Phe Pro Asn His Ser Phe
165                     170                     175

Lys His Glu Asp Pro Met Gly Gln Gln Gly Ser Leu Gly Glu Gln Gln
        180                     185                     190

Tyr Ser Val Pro Pro Pro Val Tyr Gly Cys His Thr Pro Thr Asp Ser
        195                     200                     205

Cys Thr Gly Ser Gln Ala Leu Leu Leu Arg Thr Pro Tyr Ser Ser Asp
        210                     215                     220

Asn Leu Tyr Gln Met Thr Ser Gln Leu Glu Cys Met Thr Trp Asn Gln
225                     230                     235                     240

Met Asn Leu Gly Ala Thr Leu Lys Gly Val Ala Ala Gly Ser Ser Ser
            245                     250                     255

Ser Val Lys Trp Thr Glu Gly Gln Ser Asn His Ser Thr Gly Tyr Glu
            260                     265                     270

Ser Asp Asn His Thr Thr Pro Ile Leu Cys Gly Ala Gln Tyr Arg Ile
        275                     280                     285

His Thr His Gly Val Phe Arg Gly Ile Gln Asp Val Arg Arg Val Pro
290                     295                     300

Gly Val Ala Pro Thr Leu Val Arg Ser Ala Ser Glu Thr Ser Glu Lys
305                     310                     315                     320

Arg Pro Phe Met Cys Ala Tyr Pro Gly Cys Asn Lys Arg Tyr Phe Lys
            325                     330                     335

Leu Ser His Leu Gln Met His Ser Arg Lys His Thr Gly Glu Lys Pro
            340                     345                     350

Tyr Gln Cys Asp Phe Lys Asp Cys Glu Arg Arg Phe Ser Arg Ser Asp
        355                     360                     365

Gln Leu Lys Arg His Gln Arg Arg His Thr Gly Val Lys Pro Phe Gln
        370                     375                     380

Cys Lys Thr Cys Gln Arg Lys Phe Ser Arg Ser Asp His Leu Lys Thr
385                     390                     395                     400

His Thr Arg Thr His Thr Gly Lys Thr Ser Glu Lys Pro Phe Ser Cys
            405                     410                     415

Arg Trp Pro Ser Cys Gln Lys Lys Phe Ala Arg Ser Asp Glu Leu Val
                420                 425                 430

Arg His His Asn Met His Gln Arg Asn Met Thr Lys Leu Gln Leu Ala
            435                 440                 445

Leu

449

<210> 3

<211> 8

<212> PRT

<213> Artificial Sequence

<220>

<223> Synthetic Peptide

<400> 3

Gly Ala Ser Ala Tyr Gly Ser Leu
  1                 5

<210> 4

<211> 8

<212> PRT

<213> Artificial Sequence

<220>

<223> Synthetic Peptide

<400> 4

Cys Asn Lys Arg Tyr Phe Lys Leu
  1                 5

<210> 5

<211> 9

<212> PRT

<213> Artificial Sequence

<220>

<223> Synthetic Peptide

<400> 5

```
Arg Met Phe Pro Asn Ala Pro Tyr Leu
  1                   5
<210>   6
<211>   9
<212>   PRT
<213>   Artificial   Sequence
<220>
<223>   Synthetic   Peptide
<400>   6
Tyr Ser Ser Asp Asn Leu Tyr Gln Met
  1                   5
<210>   7
<211>   9
<212>   PRT
<213>   Artificial   Sequence
<220>
<223>   Synthetic   Peptide
<400>   7
Cys Met Thr Trp Asn Gln Met Asn Leu
  1                   5
<210>   8
<211>   9
<212>   PRT
<213>   Artificial   Sequence
<220>
<223>   Synthetic   Peptide
<400>   8
Ser Leu Gly Glu Gln Gln Tyr Ser Val
  1                   5
<210>   9
<211>   9
<212>   PRT
```

```
<213>   Artificial  Sequence

<220>

<223>   Synthetic   Peptide

<400>   9

Cys Tyr Thr Trp Asn Gln Met Asn Leu
 1                   5
```